# EUROPEAN PATENT SPECIFICATION

(11) **EP 0 338 756 B1**
(45) Date of publication and mention of the grant of the patent: **20.03.1996**
(21) Application number: 89303797.8
(22) Date of filing: 17.04.1989
(51) Int. Cl.: A61K 31/35, A61K 31/38, A61K 31/335

(54) **Use of 1,2,4-trioxanes as antiparasitics and anthelmintics**
Verwendung von 1,2,4-Trioxanen als Anti-Parasitika und Anthelmintika
Utilisation de dérivés du 1,2,4-trioxane comme médicaments anti-parasitiques et anthelmintiques

(30) Priority: 20.04.1988 GB 8809367
(43) Date of publication of application: 25.10.1989
(73) Proprietor: OXACO S.A., CH-1206 Genève (CH)
(72) Inventor: Jefford, Charles W. Univ. de Genève, Dép. Chimie, CH-1121 Genève 4 (CH)
(74) Representative: Ruffles, Graham Keith

(56) References cited:
- EP-A- 0 286 316

## Description

### BACKGROUND OF THE INVENTION

The present invention relates to therapeutic methods and pharmaceutical compositions. More particularly, this invention relates to antiparasitic and other activities of 1,2,4-trioxane compounds.

Artemisinin, also known as arteannuin or qinghaosu, is active against Plasmodium falciparum, the protozoal causitive agent of malaria. The compound was isolated from the shrub Artemisia annua L (see for example Science (1985), 228, 1049), and is a 1,2,4-trioxane with the structure: 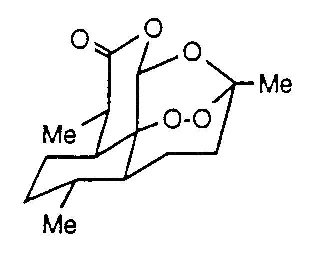
In European Patent Specification 286316 published on 12 October 1988, there are described 1,2,4-trioxane derivatives which are active as antimalarial agents. It is said that some of the derivatives have shown immunosuppressive activity. Thus the derivatives are of utility for the treatment of tropical diseases such as malaria, or for the treatment of diseases of immunological nature or viral origin.

### SUMMARY OF THE INVENTION

It has now been found that 1,2,4-trioxanes have further pharmacological activities, apart from anti-malarial activity. In particular, without being restrictive, it has been found that 1,2,4-trioxanes have anti-parasiticidal activity, embracing activity not only against other protozoa apart from Plasmodium spp, but also activity against helminths.

Accordingly, the present invention provides the use in a process for the preparation of a pharmaceutical composition for use in a parasiticidal method of therapeutic treatment, other than treatment of malaria, of a 1,2,4-trioxane which is a 3-substituted cycloalkan[e]-1,2,4-trioxane, and more especially a 3-substituted Δ⁵ - or Δ⁶-cyclopenta[e]-1,2,4-trioxane or a 3-substituted Δ⁵ - or Δ⁶-cyclohexa[e]-1,2,4-trioxane.

Particularly preferred compounds thus have the ring structure shown by formula (A) and formula (B): 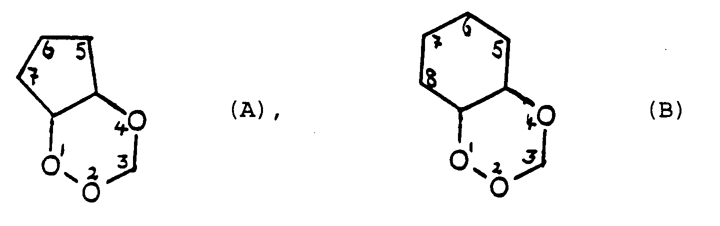
The compund is 3-substituted, and is then a 3-substituted cyclopenta[e]1,2,4-trioxane or a 3-substituted cyclohexa[e]1,2,4-trioxane, for instance a 3-substituted Δ ⁵- or Δ ⁶-6,7a-diarylcyclopenta[e]1,2,4-trioxane or a 3-substituted Δ ⁵- or Δ ⁶-6,8a-diarylcyclohexa[e]1,2,4-trioxane. Typical substituents for the 3-position comprise the groups R¹ and R² mentioned below. Such compounds may be prepared by methods available in the literature, including the method described in EP286316.

Examples of diseases which now may be treated in accordance with this invention by 1,2,4-trioxanes include toxoplasmosis, leishmaniasis, pneumonitis, and onchocerciasis.

Infection with Toxoplasma gondii, a cosmopolitan protozoan parasite, produces only benign symptoms in normal individuals. The infection, however, is a major hazard for immunocompromised patients, such as those suffering from acquired immune deficiency syndrome (AIDS), and pregnant women who become infected for the first time. A need remains for an effective drug which is less toxic than the most effective treatment currently available, either the synergistic combination of pyrimethamine with sulfonamides, or macrolide drugs such as erythromycin.

Infection with Leishmania donovani, a flagellate protozoa spread by sandflies, is the cause of Kala Azar, the systemic form of leishmaniasis affecting the blood, lymphatics, spleen and marrow.

Infection with Pneumocystis carinii, an opportunistic organism believed to be a protozoon, is the underlying cause of pneumonitis in immunocompromised hosts.

Infection with Onchocerca volvulus, a filarial worm, can lead to onchocerciasis or river blindness, which afflicts some 17 million people who live near fast-flowing water in Africa and Central and South America. It is one of the more damaging filarial parasites and effective measures are still awaited for its eradication. Furthermore, onchocerciasis in cattle remains a problem, rendering the meat unfit for human consumption.

It has now been found that certain 1,2,4-trioxanes exhibit activity against non-Plasmodium protozoal parasites, especially mastigotes, and especially parasites of the species Toxoplasma, Leishmania, and Pneumocystis, and against nematodal parasites, especially filariae, and especially parasites of the species Onchocerca. The utility extends to diseases of animals such as cattle as well as diseases in humans.

The activity is illustrated by in vitro tests using compounds in accordance with EP 286316, to which the reader is now referred. Thus, the present invention is especially directed at compositions based on 1,2,4-trioxane derivatives of formula (I): 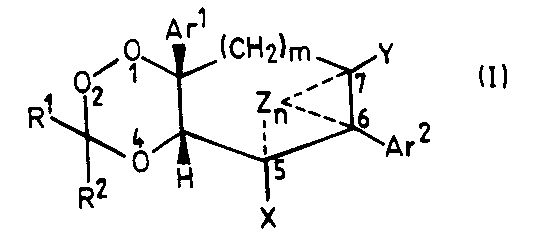
(wherein
the subscripts n and m are equal to 0 or 1;
the symbol Z represents an epoxide oxygen atom at the 5,6 or 6,7 positions, or a pair of electrons forming a double bond at the 5,6 or 6,7 positions;
each of the symbols Ar¹ and Ar², being the same or different, represents an aromatic group which is optionally substituted with one or more substituents chosen from alkyl groups, aryl groups, alkoxy groups, a hydroxy group, halogen atoms, carboxy groups, optionally alkyl-substituted amino groups or alkoxycarbonyl groups;
each of the symbols R¹ and R², being the same or different, represents a linear or branched alkyl group, which is optionally substituted with one or more substituents chosen from alkyl groups, aryl groups, alkoxy groups, a hydroxy group, halogen atoms, carboxy groups, optionally alkyl-substituted amino groups or alkoxycarbonyl groups; or R¹ and R², taken together with the carbon atom to which they are attached, form an alicyclic group which is optionally interrupted by one or more oxygen, sulphur or nitrogen atoms and which group is optionally substituted with one or more substituents chosen from alkyl groups, aryl groups, alkoxy groups, a hydroxy group, halogen atoms, carboxy groups, optionally alkyl-substituted amino groups or alkoxycarbonyl groups;
each of X and Y, being the same or different, represents a hydrogen atom or a functional group which is a hydroxy group, a peroxide group, an optionally chiral oxycarbonoyl, oxycarbonyloxy or oxyalkylcarbonyloxy ester, a carboxylic acid, a ketone, an imine, a hydrazone, an aminoacid, a peptide residue, a glycosyl group, a phosphoryl group, a diphosphenyl group, or a phosphate residue;
together with a pharmaceutically acceptable carrier.

The reader is referred to EP 286316-A1 for illustrative and preferred definitions for each of m, n, Ar¹, Ar², R¹, R², X and Y. More particularly preferred are Compounds 1 to 12 shown in the following Table 1:

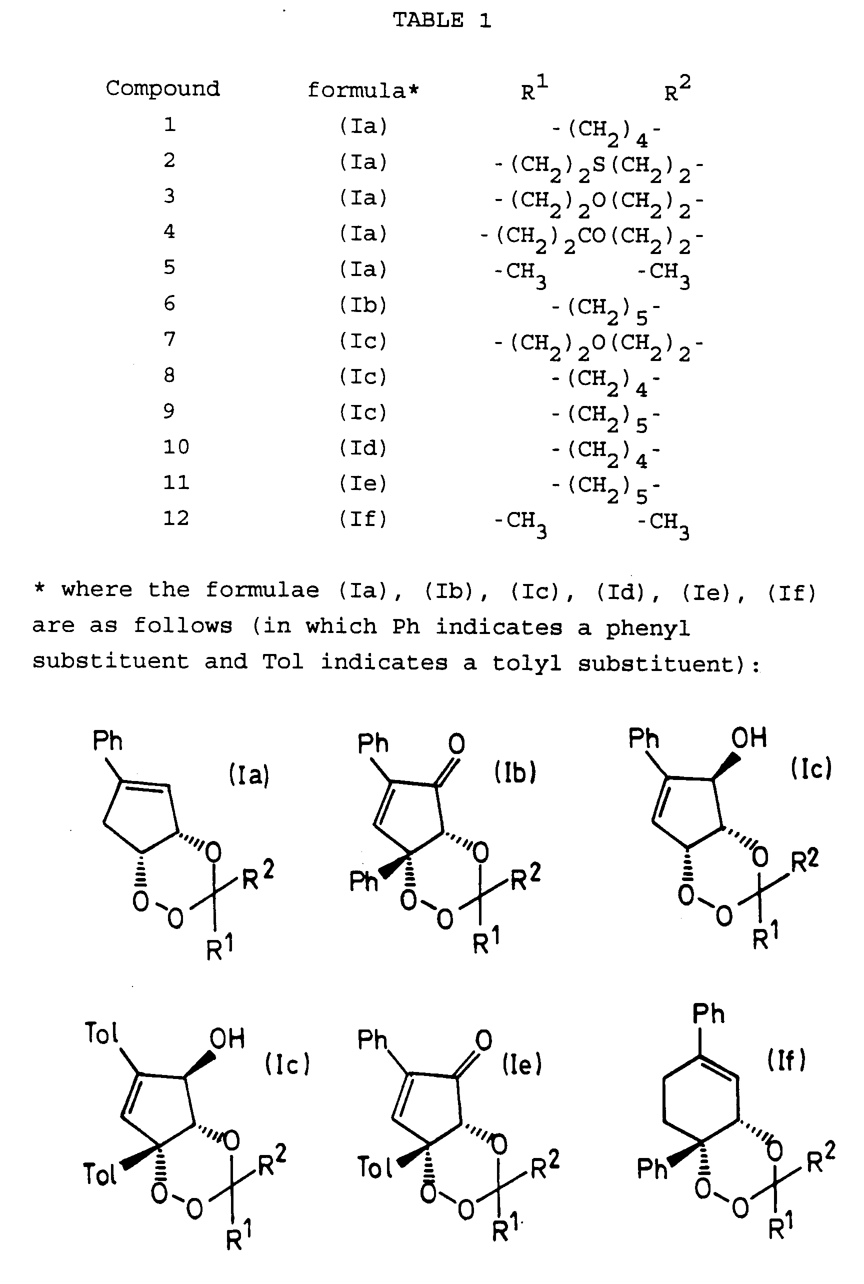

The 1,2,4-trioxane compounds for use in the present invention typically have low toxicity, permitting massive doses to be given to patients at the brink of death. For example, the compounds can be formulated as suspensions in oil and injected intramuscularly as a single shot. While formulations for parenteral administration may be used, it is within this invention to formulate the compounds as formulations for oral administration. In general, formulation of the compounds of this invention can be based on the conventional techniques available to the pharmacist. Tablets for prophylactic treatment are especially suitable. To this end, some modifications of the compounds may be desirable, for example to enhance water solubility through inclusion of hydroxy, carboxyl, amino or other functional substituents. Suitable modifications can be achieved by routine experimentation. Indeed, an advantage of the present invention is that many different compounds are available by synthetic routes.

### EXAMPLES OF THE INVENTION

### Example 1: activity against Toxoplasmosis gondii

### (a) within macrophages, assessed by radioactivity studies

Unelicited murine peritoneal macrophages obtained by washing the peritoneal cavities of Swiss-Webster mice with 3 ml of Hanks' Balanced Salt Solution (HBSS) (4°C) containing 5 U ml⁻¹ of heparin were purified by centrifugation in Ficoll-Paque (Pharmacia) 500 x g for 15 min, pooled and suspended in medium 199 (M199) containing 10% heat-inactivated (56°C for 60 min) fetal calf serum (FCS). An amount of 3 x 10⁵ cells was seeded into each well of 96-well dishes (Costar) and allowed to adhere during 2-3 h at 37°C in an 5% CO₂-95% air atmosphere. The nonadherent cells were removed by washing twice with prewarmed HBSS and challenged with 3 x 10⁵ tachyzoites of the RG strain of Toxoplasmosis gondii in M199-3% FCS for 1 h. After washing twice with HBSS, the infected monolayers received the test drugs (Compounds 1 to 10 of Table 1) at various concentrations in M199-10% FCS. The monolayers were then pulsed with 2.5 µCi of [³H]uracil (Amersham) during a period of 18 h. The acid-precipitable radioactivity on each well was counted by using a filtration procedure and the IC₉₀ was calculated by probit analysis. The results are shown in the following Table 2:

**Table 2**

| Compound no | Inhibitory concentration (IC) µg ml⁻¹ | |
|---|---|---|
| | IC₅₀ | IC₉₀ |
| 1 | 2 | 6.8 |
| 2 | 1.7 | 5.3 |
| 3 | 106 | 260 |
| 4 | 285 | >1000 |
| 5 | - | - |
| 6 | 26 | 54 |
| 7 | 580 | >1000 |
| 8 | - | - |
| 9 | 420 | 850 |
| 10 | - | - |
| (where "-" indicates not active up to 484 µg ml⁻¹) | | |

It can be seen that the Compounds 1 and 2 were the most active, with 90% inhibitory concentrations (IC₉₀) at 6.8 and 5.3 µg ml⁻¹, respectively.

### (b) within macrophages, assessed by light microcroscopy

2 x 10⁶ of the macrophage cells were seeded into each chamber of four-chamber glass slides (Lab-Tek). After removing nonadherent cells with HBSS, the monolayers were infected with 2 x 10⁶ T. gondii for 1 h followed by addition of the drugs with the media over 18 h. Monolayers were fixed with 0.4% 9-aminoacridine (50% ethanol-water vol/vol), Giemsa-stained, and assessed by light microscopy. The results are shown in the following Table 3, including results of control compounds:

**Table 3**

| Drug | (µg ml⁻¹) | % infected cells | Mean T. gondii per infected vacuole | T. gondii per 100 cells |
|---|---|---|---|---|
| none | 0 | 42 | 4.3 | 191 |
| 1 | 6.8 | 19 | 1.6 | 38 |
| 2 | 5.3 | 14 | 1.2 | 35 |
| 6 | 54 | 20 | 1.9 | 43 |
| P | 1 | 14 | 1.2 | 33 |
| P+S | 0.1+25 | 18 | 1.9 | 42 |
| P: pyrimethamine P+S: pyrimethamine in combination with sulfadiazine | | | | |

Thus, microscopic examination of the infected macrophages after treatment with compounds 1, 2 and 6 at their respective IC₉₀ values confirmed the inhibition of the intracellular growth of toxoplasma. This activity was comparable to that of pyrimethamine, and pyrimethamine in combination with sulfadiazine.

Control experiments indicated that at concentrations up to four times their IC₉₀ values the 1,2,4-trioxanes affected neither the viability of uninfected macrophages as assessed by morphological criteria and trypan blue dye exclusion test nor the viability of extracellular T. gondii, as judged by their ability to reinfect macrophages.

### (c) within HeLa cells, assessed by light microcroscopy

Confluent epithelium-like HeLa (ATCC CCL2) cell monolayers growing on 6-well plastic multidish (9.6 cm², Nunc, Roskilde, Denmark) were used. After washing with HBSS, the monolayers were infected with 3 x 10⁶ ml⁻¹ T. gondii in M199-3% FCS (total amount per well, 3 ml) for 1 h, washed twice with HBSS, and the drugs were added with M199-10% FCS over 22 h. Monolayers were then fixed, Giemsa-stained and assessed by light microscopy. The results are shown in the following Table 4:

**Table 4**

| Drug | (µg ml⁻¹) | % infected cells | Mean T. gondii per infected vacuole | T. gondii per 100 cells |
|---|---|---|---|---|
| none | 0 | 49 | 8.1 | 620 |
| 1 | 6.8 | 23 | 1.8 | 46 |
| 2 | 5.3 | 23 | 1.3 | 38 |

Since the compounds 1 and 2 were able to block parasite growth when T. gondii was harboured by cells of an epithelium-like cell line (HeLa), it follows that the antitoxoplasmic activity of the 1,2,4-trioxanes was not caused by a macrophage-specific mechanism, such as respiratory burst. Moreover, macrophages pre-incubated with different concentrations of 1,2,4-trioxanes, during 24 h, did not become activated and were not able to restrict the multiplication of T. gondii without the presence of the 1,2,4-trioxanes (data not shown).

### Example 2: activity against Onchocerca gutturosa

Testing in vitro against Onchocerca gutturosa is a valid model for activity against Onchocerca volvulus.

### (a) assessed by motility

Testing of test drugs (Compounds 1, 2, 3, 4, 5, 7, 8, 9, 11, and 12 of Table 1) against the parasitic worms Onchocerca gutturosa was carried out in accordance with the method described in J Helminth (1987) 60, 271, firstly using parasites, medium, serum, cells and test drugs.

The most striking result was shown by Compound 9, which immediately immobilized worms in less than a day. Compounds 4, 5, and 11 were also significantly effective at a day's exposure. In comparison, artemisinin was ineffective, while arteether reduced motility only by 30% after a day. On two to three days exposure, Compounds 2, 8, 9 and 12 were also markedly inhibitory. After an induction period, compounds 1 and 7 completely stopped motility, in 7 days. Thus, after 7 days, Compounds 1, 2, 4, 5, 7, 8, 9, 11 and 12 had reduced motility by 100%. Compound 3 was not so effective in this test.

These results compared favorably with those reported under the same conditions for commercial anti-helmintic drugs (see: J Helminth (1987) 60, 271).

Secondly, test drugs (Compounds 2, 3, 4, 7, 8, 9 and 12 of Table 1) were tested using parasites, medium and drug. An immediate response was elicited for Compounds 4, 8, and 9 after one day. After 2-3 days, Compounds 2, 7, and 12 had also reduced motility to less than 1, except for Compound 3 which performed poorly. However, between 4 and 7 days, all the tested Compounds had completely immobilized the worms. Under these conditions, commercial anti-helminthic drugs and artemisinin also cut motility by some 30-40%. Arteether also reduced motility to 4 and 2, after 4 and 7 days respectively.

### (b) assessed by tetrazolium colorimetric test

A suitable colorimetric assay uses formation of formazan, a blue dye, from tetrazolium cation generated by 3-[4,5-diethylthiazol-2-yl]-2,4-diphenyltetrazolium bromide (J Helminth (1987) 60, 271). Firstly, Compounds 1, 2, 3, 4, 5, 7, 8, 9, 11 and 12, along with artemisinin and arteether were tested using parasites, medium, cells and test drug. Artemisinin was ineffective, and all the test Compounds performed better than arteether, which caused about 40% inhibition of formazan formation. In fact, all these tested Compounds caused more than 50% inhibition at 5x10⁻⁵ M. Compounds 9, 11 and 12 gave more than 90% inhibition.

Secondly, Compounds 2, 3, 4, 7, 8, 9, and 12, artemisinin and arteether were tested using parasites, medium and test drugs. Arteether was ineffective, artemisinin inhibited to about 25%, and except for Compound 3, all the test Compounds inhibited formazan formation to a degree better than 80%.

### Example 3: activity against Leishmania donovani

Some of the Compounds were tested against mouse peritoneal macrophages infected with mastigotes of Leishmania donovani HU3 according to a published protocol (J Antimicrobial Chemoth (1984) 14, 463).

At 30µM, activity in the range 35 to 97% inhibition was shown by four drugs, namely Compounds 1, 2, 7 and 10. Other Compounds were less active. One drug, Compound 1, showed high activity (70% inhibition) at 10µM. This compound had an ED₅₀ of around 4.5 µm.

## Claims

1. The use in a process for the preparation of a pharmaceutical composition for use in a parasiticidal method of therapeutic treatment, other than treatment of malaria, of a 1,2,4-trioxane which is a 3-substituted cycloalkan[e]-1,2,4-trioxane, and more especially a 3-substituted Δ⁵- or Δ⁶-cyclopenta[e]1,2,4-trioxane or a 3-substituted Δ⁵- or Δ⁶-cyclohexa[e]1,2,4-trioxane.

2. The use according to claim 1, in the preparation of a pharmaceutical composition for use against protozoa apart from Plasmodium spp.

3. The use according to claim 1, in the preparation of a pharmaceutical composition for use against helminths.

4. The use according to claim 1, in the preparation of a pharmaceutical composition for use in treatment of a disease selected from toxoplasmosis, leishmaniasis, pneumonitis, and onchocerciasis.

5. The use according to any one of claims 1-4, in which the 1,2,4-trioxane is a 3-substituted Δ⁵- or Δ⁶-6,7a-diarylcyclopenta[e]1,2,4-trioxane or a 3-substituted Δ⁵- or Δ⁶-6,8a-diarylcyclohexa[e]1,2,4-trioxane.

6. The use according to claim 4 in which the 1,2,4-trioxane has the formula: 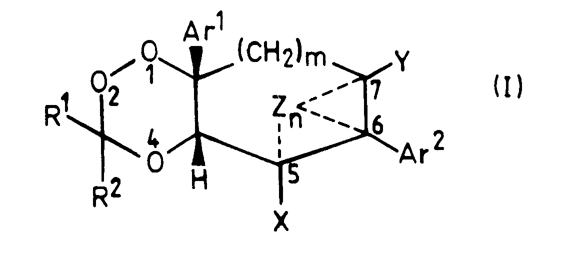 (wherein
the subscripts n and m are equal to 0 or 1;
the symbol Z represents an epoxide oxygen atom at the 5,6 or 6,7 positions, or a pair of electrons forming a double bond at the 5,6 or 6,7 positions;
each of the symbols Ar¹ and Ar², being the same or different, represents an aromatic group which is optionally substituted with one or more substituents chosen from alkyl groups, aryl groups, alkoxy groups, a hydroxy group, halogen atoms, carboxy groups, optionally alkyl-substituted amino groups or alkoxycarbonyl groups;
each of the symbols R¹ and R², being the same or different, represents a linear or branched alkyl group, which is optionally substituted with one or more substituents chosen from alkyl groups, aryl groups, alkoxy groups, a hydroxy group, halogen atoms, carboxy groups, optionally alkyl-substituted amino groups or alkoxycarbonyl groups; or R¹ and R², taken together with the carbon atom to which they are attached, form an alicyclic group which is optionally interrupted by one or more oxygen, sulphur or nitrogen atoms and which group is optionally substituted with one or more substituents chosen from alkyl groups, aryl groups, alkoxy groups, a hydroxy group, halogen atoms, carboxy groups, optionally alkyl-substituted amino groups or alkoxycarbonyl groups;
each of X and Y, being the same or different, represents a hydrogen atom or a functional group which is a hydroxy group, a peroxide group, an optionally chiral oxycarbonoyl, oxycarbonyloxy or oxyalkylcarbonyloxy ester, a carboxylic acid, a ketone, an imine, a hydrazone, an aminoacid, a peptide residue, a glycosyl group, a phosphoryl group, a diphosphenyl group, or a phosphate residue;
together with a pharmaceutically acceptable carrier.

## Patentansprüche

1. Verwendung in einem Verfahrer zur Herstellung einer pharmazeutischen Zusammensetzung zur Anwendung in einer parasitiziden Methode einer therapeutischen Behandlung, außer der Behandlung von Malaria, eines 1,2,4-Trioxans, bei dem es sich um ein 3-substituiertes Cycloalkan[e]-1,2,4-trioxan handelt und spezieller um ein 3-substituiertes Δ⁵- oder Δ⁶-Cyclopenta[e]-1,2,4-trioxan oder ein 3-substituiertes Δ⁵- oder Δ⁶-Cyclohexa[e]-1,2,4-trioxan.

2. Verwendung nach Anspruch 1 in der Herstellung einer pharmazeutischen Zusammensetzung zur Anwendung gegen Protozoen, außer Plasmodium spp.

3. Verwendung nach Anspruch 1 in der Herstellung einer pharmazeutischen Zusammensetzung zur Anwendung gegen Helminthen.

4. Verwendung nach Anspruch 1 in der Herstellung einer pharmazeutischen Zusammensetzung zur Anwendung in der Behandlung einer Erkrankung, ausgewählt aus Toxoplasmose, Leishmaniasis, Pneumonitis und Onchozerkose.

5. Verwendung nach einen der Ansprüche 1 bis 4, bei der das 1,2,4-Trioxan ein 3-substituiertes Δ⁵- oder Δ⁶-6,7a-Diarylcyclopenta[e]-1,2,4-trioxan oder ein 3-substituiertes Δ⁵- oder Δ⁶-6,8a-Diarylcyclohexa[e]-1,2,4-trioxan ist.

6. Verwendung nach Anspruch 4, bei der das 1,2,4-Trioxan die Formel hat: 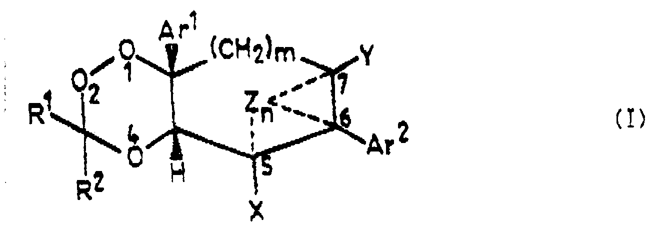 (worin sind:
jeder der Indizen m und n gleich Null oder 1;
das Symbol Z ein Epoxid-Sauerstoff-Atom an den Stellen 5,6 oder 6,7; oder ein Elektronenpaar, das an den Stellen 5,6 oder 6,7 eine Doppelbindung bildet;
jedes Ar¹ und Ar² gleich oder verschieden und stellen eine aromatische Gruppe dar, die wahlweise substituiert ist;
jedes R¹ und R² gleich oder verschieden und stellen eine gerade oder verzweigte Alkyl-Gruppe, die wahlweise substituiert ist, oder R¹ und R² bilden zusammengenommen mit dem Kohlenstoff-Atom, an dem sie gebunden sind, eine alicyclische Gruppe, die wahlweise von einen oder mehreren Sauerstoff-, Schwefel- oder Stickstoff-Atomen unterbrochen ist und welche Gruppe wahlweise mit einer oder mehreren Alkyl- oder Aryl-Gruppen unterbrochen ist, oder eine oder mehrere functionelle Gruppen; und
jedes X und Y gleich oder verschieden und stellt ein Wasserstoff-Atom oder eine functionelle Gruppe dar, die Sauerstoff, Stickstoff oder Schwefel enthält)
jedes X und Y gleich oder verschieden und stellt ein Wasserstoff-Atom oder eine funktionelle Gruppe dar, die eine Hydroxy-Gruppe, eine Peroxid-Gruppe, ein wahlweise chiraler Oxycarbonyl-, Oxycarbonyloxy- oder Oxyalkylcarbonyloxyester, eine Carbonsäure, ein Keton, ein Imin, ein Hydrazon, eine Aminosäure, ein Peptid-Rest, eine Glycosyl-Gruppe, eine Phosphoryl-Gruppe, eine Diphosphenyl-Gruppe oder ein Phosphat-Rest ist;
und zwar zusammen mit einem pharmazeutisch zulässigen Träger.

## Revendications

1. Utilisation, dans un procédé pour la préparation d'une composition pharmaceutique à utiliser dans un procédé parasiticide d'un traitement thérapeutique, autre que le traitement de la malaria, d'un 1,2,4-trioxanne qui est un cycloalcane[e]-1,2,4-trioxanne substitué en 3, et plus spécialement un Δ⁵- ou Δ⁶-cyclopenta[e]-1,2,4-trioxanne substitué en 3 ou un Δ⁵- ou Δ⁶-cyclohexa[e]-1,2,4-trioxanne substitué en 3.

2. Utilisation selon la revendication 1, dans la préparation d'une composition pharmaceutique pour l'utilisation contre des protozoaires autres que Plasmodium spp.

3. Utilisation selon la revendication 1, dans la préparation d'une composition pharmaceutique pour l'utilisation contre les helminthes.

4. Utilisation selon la revendication 1, dans la préparation d'une composition pharmaceutique pour l'utilisation dans le traitement d'une maladie choisie parmi la toxoplasmose, la leishmaniose, une pneumopathie et l'onchocercose.

5. Utilisation selon l'une quelconque des revendications 1 à 4, dans laquelle le 1,2,4-trioxanne est un Δ⁵- ou Δ⁶-6,7a-diarylcyclopenta[e]-1,2,4-trioxanne substitué en 3 ou un Δ⁵- ou Δ⁶-6,8a-diarylcyclohexa[e]-1,2,4-trioxanne substitué en 3.

6. Utilisation selon la revendication 4, dans laquelle le 1,2,4-trioxanne a la formule: 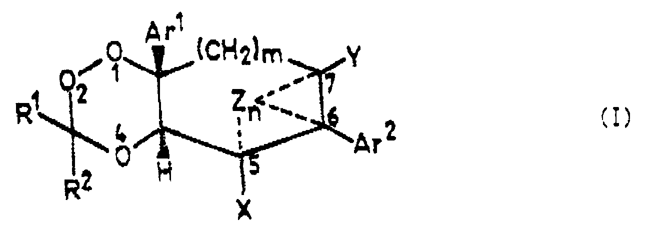 (dans laquelle
chacun des indices m et n est égal à 0 ou 1;
le symbole Z représente un atome d'oxygène d'époxyde aux positions 5,6 ou 6,7, ou une paire d'électrons formant une double liaison aux positions 5,6 ou 6,7;
chacun des symboles Ar¹ et Ar², étant les mêmes ou différents, représente un groupe aromatique qui est facultativement substitué;
chacun des symboles R¹ et R², étant les mêmes ou différents, représente un groupe alkyle linéaire ou ramifié, qui est facultativement substitué, ou bien R¹ et R², pris ensemble avec l'atome de carbone auquel ils sont attachés, forment un groupe alicyclique qui est facultativement interrompu par un ou plusieurs atomes d'oxygène, de soufre ou d'azote, ce groupe étant facultativement substitué avec un ou plusieurs groupes alkyles ou aryles, ou un ou plusieurs groupes fonctionnels; et
chacun de X et Y, étant les mêmes ou différents, représente un atome d'hydrogène ou un groupe fonctionnel qui contient de l'oxygène, de l'azote ou du soufre)
chacun de X et Y étant les mêmes ou différents, représente un atome d'hydrogène ou un groupe fonctionnel qui est un groupe hydroxy, un groupe peroxy, un ester d'oxycarbonyle, d'oxycarbonyloxy ou d'oxyalkylcarbonyloxy facultativement chiral, un acide carboxylique, une cétone, une imine, une hydrazone, un acide aminé, un résidu peptidique, un groupe glycosyle, un groupe phosphoryle, un groupe diphosphényle ou un résidu phosphate;
avec un véhicule pharmaceutiquement acceptable.
